# EUROPEAN PATENT SPECIFICATION

(11) **EP 0 845 245 B1**
(45) Date of publication and mention of the grant of the patent: **02.05.2002**
(21) Application number: 97118761.2
(22) Date of filing: 29.10.1997
(51) Int. Cl.: A61B 17/72, A61B 17/60

(54) **A locking marrow nail with adjustable openings for locking screws**
Verriegelbarer Marknagel mit verstellbaren Öffnungen für Verriegelungsschrauben
Broche médullaire verrouillable à ouvertures réglables pour vis de verrouillage

(30) Priority: 22.11.1996 DE 29620327 U
(43) Date of publication of application: 03.06.1998
(73) Proprietor: Stryker Trauma GmbH, 24232 Schönkirchen (DE)
(72) Inventor: Gahr, Ralf Herbert, Dr. med., 04448 Hohenheida (DE); Harder, Hans Erich, 24253 Probsteierhagen (DE)
(74) Representative: Patentanwälte Hauck, Graalfs, Wehnert, Döring, Siemons

(56) References cited:
- EP-A- 0 506 420
- WO-A-94/27514
- US-A- 4 628 919

## Description

The invention relates to a locking nail corresponding to the preamble of claim 1.

Marrow nails and special locking nails are known aids for osteosynthesis, which are introduced into the marrow space of the damaged bone in order mechanically bridge the bone lesion. With locking nails, locking screws which are mounted transversely through the bone and through the locking nail serve to secure the connection between the bone and locking nail against displacement in the direction of the bone axis as well as against rotation about the bone axis.

With usual locking nails up to now, locking screws are placed in discrete openings, they are arranged essentially radially in the locking nail, on the proximal thigh e.g. also at an inclined angle to the shank axis, but in each case with respect to the length of the shank as well as with respect to the angle to the shank, they are not adjustably arranged.

Due to the individual anatomical or pathological situation with various patients, in many cases it would be advantageous to deviate from the position or the angle of the opening for a locking screw and to place a locking screw elsewhere than is determined by the opening.

Accordingly this invention achieves the object of being able to adjust the openings on locking nails for the locking screws according to position and angle, in order to adapt them to the individual situation concerning different patients, without having to prepare a sortiment of complicated and expensively manufactured locking nails with differing arrangements of the openings for the locking screws.

This object is achieved with the present invention by those features formulated in claim 1.

With the help of the present invention a locking nail may be adapted before implantation to the individual anatomical or pathological situation of a patient in that for the locking screws which are to run through the locking nail at a certain position and a certain angle corresponding to this individual situation, openings are placed and adjusted at a suitable position and a suitable angle.

The locking nail according to the invention is distinguished by the fact that in at least one region of the locking nail in which locking screws are to be placed, an elongate opening radially passing through the shank of the locking nail extends in the longitudinal direction of the shank, and in which at least two sliding blocks are unrotatably guided and are secured against movement from one another. For accommodating a locking screw an opening is formed from at least one intermediate space between the sliding blocks, which is aligned to the elongate opening, so that a radially passing through opening arises for a locking screw. In a preferred embodiment of the invention the sliding surfaces of the sliding blocks, which are aligned to the intermediate space and limit the intermediate space, are concave in order to adapt themselves to the cylindrical form of the locking srews placed in this opening. The sliding blocks are guided within the shank in the elongate opening preferably by way of a positive fit. In a preferred formation of the invention they comprise radial projections which engage into the elongate opening and which essentially occludingly fill in these openings with the outer contour. Also in the longitudinal direction of the shank the elongate opening is essentially filled in by any number of sliding blocks, which are secured from their movement away from one another for example by a closure at the end of the elongate opening. In a preferred formation of the invention the closure is formed by a closure screw which also may be employed for adjusting the width of the intermediate spaces between the sliding blocks for the flush accomodation of locking screws of differing diameters; the closure screw occludes an opening, axial with respect to the shank, of the elongate opening at the end of the locking nail, wherein the elongate opening up towards this end extends over preferably a third of the length of the locking nail. At the same time the elongate opening may be located at the proximal and/or distal end of the locking nail. Every end of the locking nail provided with an elongate opening, in this preferred embodiment of the invention may be provided with a ring which surrounds the locking nail in order to prevent the spreading out of the end of the shank. With this the ring leaves the axial opening free for introducing the sliding blocks. At the same time each elongate opening, with respect to the manufacture may be preferably formed as a slot which extends in the longitudinal direction of the shank and radially passing through up to the end of the locking nail in question, wherein the shank at least in the region of the slot is formed cylindrically hollow so that the sliding blocks may be guided with a positive fit in the hollow cylindrical inner contour of the shank.

By way of the application of differing sliding blocks, any number of openings, various angles of the openings to the longitudinal direction of the shank, as well as various distances between the openings and the end of the locking nail may be arranged. With this each elongate opening is so filled in with a length and angle corresponding to the sliding blocks in the sliding surface that there arises intermediate spaces of a desired number, position and alignment.

A preferred embodiment form of the invention is described in the following by way of the appended drawings in which
- Fig. 1: shows a partial cross section in a lateral view through a shank end of a locking nail according to the invention with locking screws;
- Fig. 2: shows a partial plan view of the same end of the locking nail without locking screws, with an opened closure;
- Fig. 3: shows a cross section through the shank end taken along line A-B in Fig. 2, without sliding blocks;
- Fig. 4: shows a plan view of a sliding block in Fig. 2 in the direction of arrow C; and
- Fig. 5: shows a three-dimensional part view of the same shank end from Fig. 1 and 2 with an additional ring without locking screws, sliding blocks and closure.

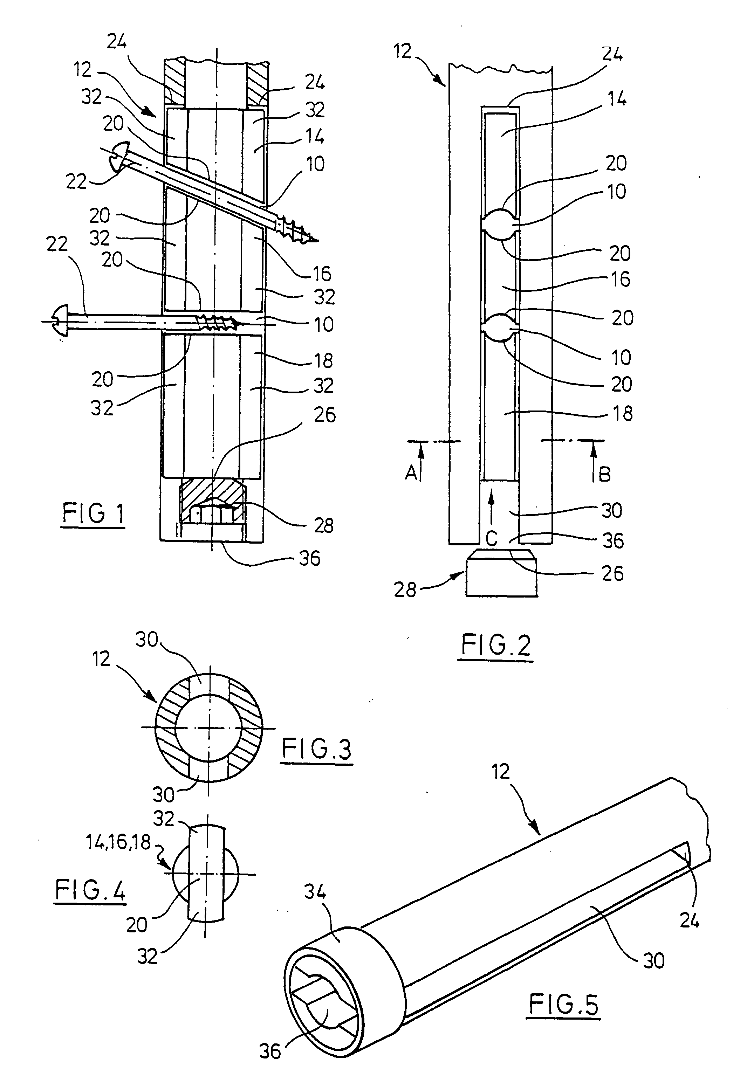

With reference to Fig. 1 and 2 firstly in Fig. 2 two openings 10 in the end of a shank 12 of a locking nail are to be seen, which are formed from the intermediate spaces between three sliding blocks 14, 16 and 18. The sliding surfaces 20 which are aligned towards the openings 10 and limit these, are concave. Fig. 1 shows in the openings 10 accommodated locking screws 22 which are guided in their direction by the sliding surfaces 20 of the sliding blocks 14, 16 and 18. The distances of the openings 10 and thus of the locking screws 22 to one another as well as to the end of the locking nail is fixed by the length of the sliding blocks 14, 16 and 18. The angle of the openings 10 or of the locking screws 22 is determined by the angle of the sliding surfaces 20. With this the sliding blocks are secured against movement away from one another by stops 24 and by an adjustable stop of a closure screw 28.

With reference to Fig. 3 and 4, in Fig. 3 the hollow cylindrical shape of the shank 12 can be seen, in whose radially passing through, elongate axial opening 30 the radial projections 32 of the sliding blocks 14, 16 or 18 are accomodated and the sliding blocks are thus unrotatably guided by way of a positive fit.

Again referring to Fig. 1 and 2 the elongate opening 30 extends from the stops 24 in the longitudinal direction of the shank up to its end 36. With this Fig. 1 shows that the radial projections 32 of the sliding blocks 14, 16 and 18 essentially occlude with the outer contour of the shank 12. The elongate opening 30 is essentially filled in by the sliding blocks 14, 16 and 18 in the longitudinal direction of the shank 12. The width of the intermediate spaces between the sliding blocks 14, 16 and 18 may be adjusted by turning the closure screw 28 upwards or downwards in the longitudinal direction of the shank 12.

Fig. 2 shows that the closure screw 28 is rotated out from the end of the shank 12 so that the sliding blocks 14, 16 and 18 may be exchanged with another set of sliding blocks (not shown) with the same cross section as is shown in Fig. 4. By way of the fact that with the second set of sliding blocks the intermediate spaces between the sliding surfaces are located at a different distance to one another and to the end of the shank 12, and the sliding surfaces are aligned at a different angle to the longitudinal direction of the shank 12, the openings 10 with respect to position and angle in the shank 12 may be adjusted by way of the geometry (length between the sliding surfaces, angle of the sliding surfaces to the longitudinal axis of the shank) of various sets of sliding blocks.

Fig. 5 shows a ring 34 which surrounds the end of the shank 12 which is provided with an elongate opening 30, in order to prevent the spreading out of the shank end. With this the ring leaves an axial opening 36 free for introducing the sliding blocks 14, 16 and 18 with a cross section profile according to Fig. 4.

## Claims

1. A locking nail for placing in the marrow space of tubular bones for the care of bone fractures, with an elongate, essentially cylindrical shank (12) with a number of essentially radial openings (10) for accommodating locking screws (22), **characterised in that** at least one radial, elongate opening (30) extends in the longitudinal direction of the shank (12) and that at least two sliding blocks (14, 16, 18) are unrotatably guided within the shank (12) and are secured against a movement away from one another so that at least one opening (10) at an angle to the longitudinal direction is formed from at least one intermediate space between the sliding blocks (14, 16, 18), wherein this opening (10) is aligned to the elongate opening for accommodating a locking screw.

2. A locking nail according to claim 1, **characterised in that** the sliding surfaces of the sliding blocks (14, 16, 18), aligned to the intermediate space, are concave.

3. A locking nail according to claim 1 or 2, **characterised in that** the sliding blocks (14, 16, 18) are so formed within the shank (6) that they are secured against radially falling out of the elongate opening (10) by way of a positive fit.

4. A locking nail according to one of claims 1 to 3, **characterised in that** the sliding blocks (14, 16, 18) comprise radial projections (32) engaging into the elongate opening which occlude essentially with the outer contour of the shank (12).

5. A locking nail according to one of claims 1 to 4, **characterised by** a set of exchangeable sliding blocks (14, 16, 18) with a different length in the longitudinal direction of the shank (12) and/or a different angle between the sliding surface (20) and the longitudinal direction of the shank (12).

6. A locking nail according to one of claims 1 to 5, **characterised in that** the elongate opening (30) is essentially filled in by any number of sliding blocks (14, 16, 18) of the same or different lengths.

7. A locking nail according to one of claims 1 to 6, **characterised in that** the sliding blocks (14, 16, 18) are secured against a movement away from one another by way of a closure (28) at the end of the locking nail.

8. A locking nail according to claim 7, **characterised by** a closure screw as a closure (28).

9. A locking nail according to one of claims 1 to 8, **characterised in that** the width of the intermediate space between the sliding blocks (14, 16, 18) can be set by adjusting at least one sliding block by way of an adjustable stop (26).

10. A locking nail according to one of claims 1 to 9, **characterised in that** the length of the elongate opening (30) is at least one third of the length of the locking nail.

11. A locking nail according to claim one of claims 1 to 10, **characterised in that** an elongate opening (30) is located at the proximal and/or distal end of the locking nail.

12. A locking nail according to one of claims 1 to 11, **characterised in that** the elongate opening (30) at at least one end of the locking nail is formed by a slot which extends in the longitudinal direction of the shank (12) and extends radially passing through to the shank up to the mentioned end, wherein the shank at least in the region of the slot is formed cylindrically hollow and wherein the sliding blocks (14, 16, 18) are guided in the cylindrically hollow shank (12) by a positive fit.

13. A locking nail according to claim 12, **characterised by** a ring (30) at the end of the locking nail provided with a slot, wherein the ring surrounds the locking nail and leaves free an axial opening (36) for introducing the sliding blocks (14, 16, 18).

## Patentansprüche

1. Verriegelungsnagel zum Einbringen in den Markraum von Röhrenknochen zur Versorgung von Knochenfrakturen mit einem länglichen, im wesentlichen zylindrischen Schaft (12) mit einer Anzahl im wesentlichen radialer Öffnungen (10) zum Aufnehmen von Verriegelungsschrauben (22), **dadurch gekennzeichnet, daß** mindestens eine radiale, längliche Öffnung (30) sich in Längsrichtung des Schafts (12) erstreckt und daß mindestens zwei Kulissensteine (14, 16, 18) innerhalb des Schafts (12) unverdrehbar geführt und gegen eine Bewegung voneinander fort gesichert sind, so daß mindestens eine Öffnung (10) im Winkel zur Längsrichtung aus mindestens einem Zwischenraum zwischen den Kulissensteinen (14, 16, 18) gebildet ist, wobei diese Öffnung (10) zur länglichen Öffnung zum Aufnehmen einer Verriegelungsschraube ausgerichtet ist.

2. Verriegelungsnagel nach Anspruch 1, **dadurch gekennzeichnet, daß** die zum Zwischenraum gerichteten Kulissenflächen (20) der Kulissensteine (14, 16, 18) konkav sind.

3. Verriegelungsnagel nach Anspruch 1 oder 2, **dadurch gekennzeichnet, daß** die Kulissensteine (14, 16, 18) innerhalb des Schaftes (6) so ausgebildet sind, daß sie durch Formschluß gegen Herausfallen radial aus der länglichen Öffnung (30) gesichert sind.

4. Verriegelungsnagel nach einem der Ansprüche 1 bis 3, **dadurch gekennzeichnet**, die Kulissensteine (14, 16, 18) in die längliche Öffnung eingreifende radiale Ansätze (32) aufweisen, die im wesentlichen mit der Außenkontur des Schafts (12) abschließen.

5. Verriegelungsnagel nach einem der Ansprüche 1 bis 4, **gekennzeichnet durch** einen Satz auswechselbarer Kulissensteine (14, 16, 18) mit unterschiedlicher Länge in Längsrichtung des Schafts (12) und/oder unterschiedlichem Winkel zwischen der Kulissenfläche (20) und der Längsrichtung des Schafts (12).

6. Verriegelungsnagel nach einem der Ansprüche 1 bis 5, **dadurch gekennzeichnet, daß** die längliche Öffnung (30) durch eine beliebige Anzahl Kulissensteine (14, 16, 18) gleicher oder unterschiedlicher Länge im wesentlichen ausgefüllt ist.

7. Verriegelungsnagel nach einem der Ansprüche 1 bis 6, **dadurch gekennzeichnet, daß** die Kulissensteine (14, 16, 18) durch einen Verschluß (28) am Ende des Verriegelungsnagels gegen eine Bewegung voneinander fort gesichert sind.

8. Verriegelungsnagel nach Anspruch 7, **gekennzeichnet durch** eine Verschlußschraube als Verschluß (28).

9. Verriegelungsnagel nach einem der Ansprüche 1 bis 8, **dadurch gekennzeichnet, daß** die Weite des Zwischenraums zwischen zwei Kulissensteinen 14, 16, 18) durch Verstellung mindestens eines Kulissensteins mit Hilfe eines verstellbaren Anschlags (26) einstellbar ist.

10. Verriegelungsnagel nach einem der Ansprüche 1 bis 9, **dadurch gekennzeichnet, daß** die Länge der länglichen Öffnung (30) mindestens ein Drittel der Länge des Verriegelungsnagels beträgt.

11. Verriegelungsnagel nach einem der Ansprüche 1 bis 10, **dadurch gekennzeichnet, daß** sich eine längliche Öffnung (30) am proximalen und/oder distalen Ende des Verriegelungsnagels befindet.

12. Verriegelungsnagel nach einem der Ansprüche 1 bis 11, **dadurch gekennzeichnet, daß** die längliche Öffnung (30) an mindestens einem Ende des Verriegelungsnagels durch einen Schlitz gebildet ist, der sich in Längsrichtung des Schafts (12) und radial durchgängig zum Schaft bis zum genannten Ende erstreckt, wobei der Schaft mindestens im Bereich des Schlitzes hohlzylindrisch ausgebildet ist und wobei die Kulissensteine (14, 16, 18) durch Formschluß im hohlzylindrischen Schaft (12) geführt sind.

13. Verriegelungsnagel nach Anspruch 12, **gekennzeichnet durch** einen Ring (30) am mit einem Schlitz versehenen Ende des Verriegelungsnagels, wobei der Ring den Verriegelungsnagel umfängt und eine axiale Öffnung (36) zum Einführen der Kulissensteine (14, 16, 18) frei läßt.

## Revendications

1. Clou de blocage destiné à un placement dans l'espace médullaire d'os tubulaires en vue du traitement de fractures osseuses, présentant une tige allongée, essentiellement cylindrique (12) comprenant un certain nombre d'ouvertures essentiellement radiales (10) destinées à recevoir des vis de blocage (22), **caractérisé en ce qu'**au moins une ouverture radiale, allongée (30) s'étend dans la direction longitudinale de la tige (12) et **en ce qu'**au moins deux blocs coulissants (14, 16, 18) sont guidés sans possibilité de rotation à l'intérieur de la tige (12) et sont fixés vis-à-vis d'un déplacement à l'écart les uns des autres de sorte qu'au moins une ouverture (10) à un certain angle par rapport à la direction longitudinale soit formée à partir d'au moins un espace intermédiaire entre les blocs coulissants (14, 16, 18), dans lequel cette ouverture (10) est alignée par rapport à l'ouverture allongée en vue de recevoir une vis de blocage.

2. Clou de blocage selon la revendication 1, **caractérisé en ce que** les surfaces coulissantes des blocs coulissants (14, 16, 18), alignées par rapport à l'espace intermédiaire, sont concaves.

3. Clou de blocage selon la revendication 1 ou 2, **caractérisé en ce que** les blocs coulissants (14, 16, 18) sont ainsi formés à l'intérieur de la tige (12) qu'ils sont garantis vis-à-vis d'une sortie radiale de l'ouverture allongée (10) au moyen d'un ajustement positif.

4. Clou de blocage selon l'une des revendications 1 à 3, **caractérisé en ce que** les blocs coulissants (14, 16, 18) comprennent des saillies radiales (32) venant en prise dans l'ouverture allongée, lesquelles obturent essentiellement le contour extérieur de la tige (12).

5. Clou de blocage selon l'une des revendications 1 à 4, **caractérisé par** un ensemble de blocs coulissants échangeables (14, 16, 18) présentant une longueur différente dans la direction longitudinale de la tige (12) et/ou un angle différent entre la surface coulissante (20) et la direction longitudinale de la tige (12).

6. Clou de blocage selon l'une des revendications 1 à 5, **caractérisé en ce que** l'ouverture allongée (30) est essentiellement remplie par un nombre quelconque de blocs coulissants (14, 16, 18) de longueurs identiques ou différentes.

7. Clou de blocage selon l'une des revendications 1 à 6, **caractérisé en ce que** les blocs coulissants (14, 16, 18) sont garantis vis-à-vis d'un déplacement à l'écart les uns des autres au moyen d'une fermeture (28) à l'extrémité du clou de blocage.

8. Clou de blocage selon la revendication 7, **caractérisé par** une vis de fermeture en tant que fermeture (28).

9. Clou de blocage selon l'une des revendications 1 à 8, **caractérisé en ce que** la largeur de l'espace intermédiaire entre les blocs coulissants (14, 16, 18) peut être réglée en ajustant au moins un bloc coulissant au moyen d'une butée réglable (26).

10. Clou de blocage selon l'une des revendications 1 à 9, **caractérisé en ce que** la longueur de l'ouverture allongée (30) est d'au moins un tiers de la longueur du clou de blocage.

11. Clou de blocage selon l'une des revendications 1 à 10, **caractérisé en ce qu'**une ouverture allongée (30) est située au niveau de l'extrémité proximale et/ou distale du clou de blocage.

12. Clou de blocage selon l'une des revendications 1 à 11, **caractérisé en ce que** l'ouverture allongée (30) au niveau d'au moins une extrémité du clou de blocage est formée par une fente qui s'étend dans la direction longitudinale de la tige (12) et qui s'étend radialement en passant au travers de la tige jusqu'à l'extrémité mentionnée, où la tige au moins dans la région de la fente est formée de façon creuse et cylindrique et où les blocs coulissants (14, 16, 18) sont guidés dans la tige creuse cylindrique (12) par un ajustement positif.

13. Clou de blocage selon la revendication 12, **caractérisé par** une bague (30) à l'extrémité du clou de blocage muni d'une fente, où la bague entoure le clou de blocage et laisse libre une ouverture axiale (36) en vue de l'introduction des blocs coulissants (14, 16, 18).
